# FASCICULE DE BREVET EUROPEEN

(11) **EP 0 986 585 B1**
(45) Date de publication et mention de la délivrance du brevet: **26.03.2003**
(21) Numéro de dépôt: 98925783.7
(22) Date de dépôt: 26.05.1998
(51) Int. Cl.: C08F 8/40, C07F 9/535

(54) **UTILISATION D'YLURES DE PHOSPHORE EN TANT QUE BASES FORTES FAIBLEMENT NUCLEOPHILES**
PHOSPHOR YLIDE, HERSTELLUNG UND VERWENDUNG ZU SCHWACH-NUKLEOPHILEN STARK-BASISCHEN VERBINDUNGEN
USE OF YLIDES OF PHOSPHORUS AS SLIGHTLY NUCEOPHILIC STRONG BASES

(30) Priorité: 26.05.1997 EP 97401142
(43) Date de publication de la demande: 22.03.2000
(73) Titulaire: SOCIETE DE CONSEILS DE RECHERCHES ET D'APPLICATIONS SCIENTIFIQUES (S.C.R.A.S.), 75016 Paris (FR); CENTRE NATIONAL DE LA RECHERCHE SCIENTIFIQUE, 75016 Paris Cédex (FR)
(72) Inventeur: BERTRAND, Guy, F-31320 Pechbusque (FR); BIGG, Dennis, F-91190 Gif-sur-Yvette (FR); CAZAUX, Jean-Bernard, F-30390 Aramon (FR); GOUMRI, Stéphanie Bâtiment A - Appartement A 36, F-31400 Toulouse (FR); GUERRET, Olivier, F-31400 Toulouse (FR)
(74) Mandataire: Bourgouin, André
(86) Numéro de dépôt international: FR9801048
(87) Numéro de publication internationale: WO98054229

(56) Documents cités:
- EP-A- 0 030 516
- EP-A- 0 744 393
- DE-A- 2 022 489
- FR-A- 2 019 198
- US-A- 3 274 228
- US-A- 3 681 428
- US-A- 3 725 365
- US-A- 3 919 126
- US-A- 4 043 948

## Description

La présente invention concerne l'utilisation d'ylures de phosphore en tant que bases fortes faiblement nucléophiles. L'invention concerne également des ylures de phosphore et leur préparation.

Les bases organiques fortes sont généralement nécessaires pour fonctionnaliser des molécules organiques ou organométalliques ; et plus la base est forte, mieux elle piège les protons. Afin de limiter les réactions parasites, il sera préférable et même souhaitable de choisir une base peu nucléophile. Par ailleurs, la facilité de séparer l'acide obtenu du milieu réactionnel est déterminante dans le but de simplifier les étapes de purifications.

Des ylures de phosphore ont été étudiés par Wittig dans les années 50 (Johnson, A. W., 1993, Series, Editor, *Ylides and Imines of phosphorus,* Wiley & Wiley : New York). Ces molécules, assez simples, sont utilisées pour la réaction de Wittig où leur forte nucléophilie est mise en jeu. Mais ces composés n'ont jamais été utilisés en tant que base en synthèse organique, et *a fortiori* en tant que base forte peu nucléophile.

Les bases organiques neutres fortes connues à ce jour sont essentiellement les éponges à protons (Alder, R. et al., *J. Chem. Soc. Chem. Commun.,* 1968, 723), les guanidines, les phosphatranes et les polyphosphazènes.

Les guanidines constituent la classe de bases fortes organiques la plus utilisée en synthèse organique. Les plus connues sont DBN (1,5-diazabicyclo [4.3.0] non-5-ène) et DBU (1,8-diazabicyclo [4.3.0] undec-5-ène). D'autres guanidines sont également mentionnées dans la littérature (Oediger, H. et al., *Synthesis*, 1972, 593 ; Schwesinger, R., *Angew. Chem. Int. Ed. Engl.*, 1987, 26, 1164). Ces bases trouvent leurs applications dans des réactions d'élimination ou dans des processus d'oligomérisation ou de polymérisation en tant qu'initiateur de réaction (Oediger, H. et al., *Synthesis*, 1972, 593 ; Wöhrle, D. et al., *Dyes and Pigments,* 1992, 18, 91).

Les phosphatranes sont des composés plus récents (Verkade, J., 1991, *Phosphatranes as proton abstracting reagents*, US patent n°5051533). Leur basicité est très supérieure aux guanidines, ce qui étend leur champ d'application. L'inconvénient de telles phosphines réside dans leur difficulté d'obtention et leur purification.

Quant aux polyphosphazènes, récemment mises au point par Schwesinger, ce sont les molécules organiques les plus basiques montrant une très faible nucléophilie. Elles sont cependant très difficiles à synthétiser et leur coût est très élevé (Schwesinger, R. et al., *Liebigs Ann*., 1996, 1055 ; Schwesinger, R. et al., *Angew. Chem. Int. Ed. Engl.,* 1991, 30, 1372 ; Schwesinger, R. et al., *Angew. Chem. Int. Ed. Engl.,* 1987, 26, 1167 ; Schwesinger, R. et al., *Angew. Chem. Int. Ed. Engl.,* 1987, 26, 1165 ; Schwesinger, R, *Chimia, 1985,39,269*). Leurs applications sont très variées : elles vont de la simple élimination à la benzylation d'oligopeptides (Schwesinger, R., *Chimia,* 1985, 39, 269 ; Pietzonka, T. et al., *Angew. Chem. Int. Ed. Engl*., 1992, 31, 1481). La basicité de ces molécules est suffisante pour arracher des protons peu acides et leur caractère organique libère des effets de sels gênants dans certaines alkylations (Pietzonka, T. et al., *Chem. Ber.,* 1991, 124, 1837).

Les bases alcalines telles que BuLi, LiHMDS, NaHMDS, LDA représentent également une autre grande famille de bases. Elles sont souvent utilisées avec de bons rendements pour fonctionnaliser des lactames, des cétones ou des bases de Schiff (Stork, G. et al., *J. Org. Chem.,* 1976, 41, 3491 ; Myers, A. et al., *J. Am. Chem. Soc.,* 1994, 116, 9361 ; Myers, A. et al., *J. Am. Chem. Soc.,* 1995, 117, 8488 ; Yaozhong, J. et al., *Synth. Commun*., 1990, 20, 15 ; Butcher, J., Liverton, N., Selnick, H., Helliot, J., Smith, G., et al., *Tet. Lett*., 1996, 37, 6685 ; Davis, F., Sheppard, A. et al., *J. Am. Chem. Soc.,* 1990). Les effets de sels sont très fréquents et varient souvent d'une réaction à l'autre et ces bases conservent un fort caractère nucléophile même à basse température. (Meyers, A. Kunnen, K., *J. Am. Chem. Soc.,* 1987, 109, 4405).

Le problème est donc de trouver des bases fortes peu nucléophiles, faciles à synthétiser et à moindre coût, qui se substitueraient d'une part aux bases communément employées trop nucléophiles et d'autre part aux bases récentes trop onéreuses.

L'invention concerne donc l'utilisation de produits de formule générale I' 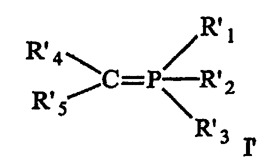 dans laquelle
R'₁, R'₂ et R'₃ représentent, indépendamment, un radical alkoxy en C₁-C₆ ou amino de formule R'R"N dans laquelle R' et R" représentent indépendamment l'un des radicaux non-substitués ou substitués (par un ou plusieurs substituants identiques ou différents) suivants : alkyle en C₁-C₆, alkoxy en C₁-C₆, cycloalkyle hydrocarboné cyclique non aromatique en C₃-C₁₀, aryl-(C₁-C₆)alkyle, aryle, dans lesquels le substituant est un atome de fluor, de chlore, de brome ou d'iode ou un radical alkyle en C₁-C₆, alkoxy en C₁-C₆, cyano, nitro ou di(C₁-C₆alkyl)amino avec les deux radicaux alkyles identiques ou différents, et les radicaux aryles étant insaturés, monocycliques ou constitués de cycles condensés, carbocycliques ou hétérocycliques, étant entendu que les radicaux hétérocycliques peuvent inclure un ou plusieurs hétéroatomes identiques ou différents choisis parmi l'oxygène, le soufre ou l'azote ;
R'₄ représente l'atome d'hydrogène, un radical alkyle en C₁-C₆ ou alkoxy en C₁-C_{6;}
R'₅ représente l'atome d'hydrogène, un radical alkyle en C₁-C₆, alkoxy en C₁-C₆ ou un support polymérique ;
en tant que base forte faiblement nucléophile.

Dans les définitions indiquées ci-dessus, l'expression halogène représente un atome de fluor, de chlore, de brome ou d'iode, de préférence chlore ou brome. L'expression alkyle inférieur représente un radical alkyle ayant de 1 à 6 atomes de carbone linéaire ou ramifié et en particulier un radical alkyle ayant de 1 à 4 atomes de carbone tels que les radicaux méthyle, éthyle, propyle, isopropyle, butyle, isobutyle, sec-butyle et tert-butyle.

Les radicaux alkoxy inférieurs peuvent correspondre aux radicaux alkyle indiqués ci-dessus. Parmi les radicaux linéaires, on peut citer les radicaux méthoxy, éthoxy, propyloxy, n-butyloxy ou n-hexyloxy. Parmi les radicaux alkoxy ramifiés, on peut citer les radicaux isopropyloxy, sec-butyloxy, isobutyloxy, ter-butyloxy, isopentyloxy, neopentyloxy ou ter-pentyloxy.

Le terme cycloalkyle inclut tout fragment hydrocarboné cyclique non-aromatique ayant de 3 à 10 atomes de carbones et de préférence cyclopropyle, cyclobutyle, cyclopentyle ou cyclohexyle.

Le terme aryle désigne les radicaux insaturés, monocycliques ou constitués de cycles condensés, carbocycliques ou hétérocycliques, étant entendu que les radicaux hétérocycliques peuvent inclure un ou plusieurs hétéroatomes identiques ou différents choisis parmi l'oxygène, le soufre ou l'azote. Des exemples de tels groupes incluent les radicaux phényle, thiényle, furyle, pyridyle, pyrimidyle, pyrrolyle, thiazolyle, isothiazolyle, diazolyle, triazolyle, thiatriazolyle, oxazolyle, benzothiényle, benzofuryle, benzopyrrolyle, benzimidazolyle, benzaxozolyle, indolyle, purinyle, naphtyle, thionaphtyle, phénanthrényle, anthracényle, biphényle, indényle, quinolyle, isoquinolyle ou quinolizinyle. Les radicaux arylalkyles inférieurs désignent les radicaux dans lesquels respectivement les radicaux aryle et alkyle inférieur sont tels que définis ci-dessus comme par exemple benzyle, phenéthyle ou naphtylméthyle.

Le terme dialkylamino représente le radical amino substitué par deux radicaux alkyles, identiques ou différents, tels que définis ci-dessus, comme par exemple diméthylamino, (méthyl)(éthyl)amino, diéthylamino.

L'expression faiblement nucléophile signifie non nucléophile envers d'autres centres que des protons. Le terme base forte correspond au terme communément utilisé par l'homme de fart dans le domaine technique considéré. Le support polymérique peut être, par exemple, de type méthacrylique, acrylique ou styrénique.

L'invention concerne plus particulièrement l'utilisation, en tant que base forte faiblement nucléophile, de produits de formule générale I' telle que définie ci-dessus caractérisée en ce que R'₁, R'₂ et R'₃ représentent, indépendamment, un radical amino de formule RR'N telle que définie ci-dessus. De préférence, R'₁, R'₂ et R'₃ représentent, indépendamment, le radical diméthylamino, (méthyl)(éthyl)amino ou diéthylamino.

L'invention concerne plus particulièrement l'utilisation, en tant que base forte faiblement nucléophile, de produits de formule générale I' telle que définie ci-dessus caractérisée en ce que R'₄ représente l'atome d'hydrogène ou un radical alkyle. De préférence, R'₄ représente l'atome d'hydrogène ou le radical méthyle ou éthyle.

L'invention concerne plus particulièrement également l'utilisation, en tant que base forte faiblement nucléophile, de produits de formule générale I' telle que définie ci-dessus caractérisée en ce que R'₅ représente un radical alkyle, et de préférence méthyle, ou un support polymérique. De manière préférentielle, le support polymérique R'₅ est de type méthacrylique, acrylique tel que le polymère expansive®, ou polystyrénique. De préférence, le support polymérique de type polystyrénique est de formule générale (s) 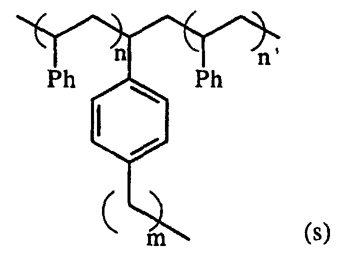 dans laquelle n, n', m sont des entiers supérieurs ou égaux à un.

Le support polymérique de formule (s) peut provenir du polymère correspondant de formule (p) 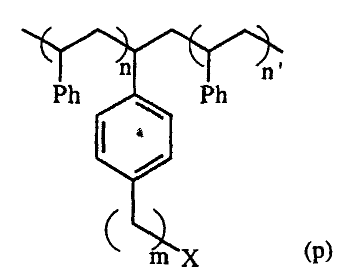 dans laquelle n, n', m sont des entiers supérieurs ou égaux à un et X représente un groupe partant. En tant que groupe partant, X peut être, par exemple, un atome d'halogène, le radical oxycarbonyle, oxysulfonyle ou oxyboronyle. De préférence, le support polymérique R'₅ provient d'un polymère polystyrénique de formule générale (p) telle que définie ci-dessus dans laquelle X représente l'atome de chlore et m est égal à 1, et plus particulièrement de la résine de Merrifield.

L'invention a plus particulièrement pour objet l'utilisation des composés de formule I' telle que définie ci-dessus, en tant que base forte faiblement nucléophile dans les réactions de N-alkylation telles que, par exemple, les réactions de N-alkylation des lactames, des succinimides, d'oligopeptides et de benzodiazépines.

L'invention a plus particulièrement pour objet également l'utilisation des produits de formule générale I' telle que définie ci-dessus en tant que-base forte faiblement nucléophile dans les réactions de C-alkylation telles que, par exemple, les réactions de C-alkylation des lactames, des succinimides, de bases de Schiff et de benzodiazépines.

L'invention a plus particulièrement pour objet l'utilisation des produits répondant aux formules suivantes :
- copolymère styrène/divinylbenzène-tris(diméthylamino)méthylènephosphorane ;
- tris(diméthylamino)-C-diméthylméthylène phosphorane.
en tant que base forte faiblement nucléophile, et notamment dans les réactions de N-alkylation ou de C-alkylation.

Parmi les composés de formule I' tels que définis, certains sont connus (Johnson, A. W., 1993, Series, Editor, *Ylides and Imines of phosphorus*, Wiley & Wiley : New York).

L'invention a donc également pour objet de ylures de phosphore et plus particulièrement les produits de formule générale I 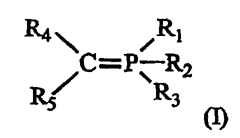 dans laquelle R₁, R₂ et R₃ représentent, indépendamment, un radical alkoxy en C₁-C₆ ou amino de formule R'R"N dans laquelle R' et R" représentent indépendamment l'un des radicaux non substitués ou substitués (par un ou plusieurs substituants identiques ou différents) suivants : alkyle en C₁-C₆, alkoxy en C₁-C₆, cycloalkyle hydrocarboné cyclique non aromatique en C₃-C₁₀, aryl-(C₁₋C₆)alkyle, aryle, dans lesquels le substituant est un atome de fluor, de chlore, de brome ou d'iode ou un radical alkyle en C₁-C₆, alkoxy en C₁-C₆, cyano, nitro ou di(C₁-C₆alkyl)amino avec les deux radicaux alkyles identiques ou différents, et les radicaux aryles étant insaturés, monocycliques ou constitués de cycles condensés, carbocycliques ou hétérocycliques, étant entendu que les radicaux hétérocycliques peuvent inclure un ou plusieurs hétéroatomes identiques ou différents choisis parmi l'oxygène, le soufre ou l'azote ;
R₄ représente l'atome d'hydrogène, un radical alkyle en C₁-C₆ ou alkoxy en C₁-C₆ ; et
R₅ représente un support polymérique.

L'invention a plus particulièrement pour objet les produits de formule générale I telle que définie ci-dessus caractérisée en ce que R₁, R₂ et R₃ représentent, indépendamment, un radical amino de formule RR'N telle que définie ci-dessus. De préférence, R₁, R₂ et R₃ représentent, indépendamment, le radical diméthylamino, (méthyl)(éthyl)amino, diéthylamino.

L'invention a plus particulièrement pour objet les produits de formule générale I telle que définie ci-dessus caractérisée en ce que R₄ représente l'atome d'hydrogène.

L'invention a plus particulièrement pour objet les produits de formule générale I telle que définie ci-dessus caractérisée en ce que le support polymérique R₅ est de type méthacrylique, acrylique tel que le polymère expansine®, ou polystyrénique. De préférence, le support polymérique de type polystyrénique est de formule générale (s) telle que définie ci-dessus.

L'invention a également pour objet un procédé de préparation des produits de formule générale I telle que définie ci-dessus, caractérisé en ce que
l'on fait réagir une phosphine de formule générale (1)

PR₁R₂R₃ (1)

dans laquelle R₁, R₂ et R₃ ont la signification indiquée ci-dessus, avec un composé de formule générale (2) 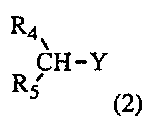 dans laquelle R₄ et R₅ ont la signification indiquée ci-dessus et Y est tel que Y⁻ représente tout anion, pour obtenir le produit de formule générale (3) 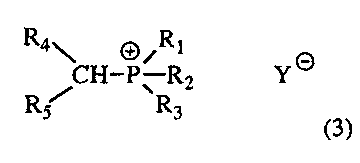 produit (3) ainsi obtenu que l'on traite avec une base forte pour obtenir un produit de formule I.

La préparation du composé (3), à partir des composés (1) et (2) s'effectue de préférence sous atmosphère inerte, comme par exemple sous atmosphère d'argon, dans un solvant polaire tel que 1,2-dichloroéthane, l'acétonitrile, le diméthoxyéthane ou le diéthoxyméthane. Le composé (2) est en général en excès stoechiométrique par rapport au composé (1). Dans ce composé (2), Y est tel que Y⁻ représente tout anion connu de l'homme de l'art tel que, par exemple, halogénures, triflate. Le milieu réactionnel est alors agité au reflux du solvant. Après les étapes classiques de lavage, le composé (3) est séché sous vide. Afin d'obtenir le produit (I), le produit (3) ainsi obtenu est traité, à température ambiante, en présence d'une base forte. La base forte peut être par exemple une base lithiée telle les lithiens d'alkyle, et plus particulièrement le butyllithium, un hydrure de sodium ou de potassium, un amidure ou un alcoolate. Le solvant peut être choisi parmi le pentane, l'éther, le tétrahydrofurane, le diméthoxyéthane ou le diéthoxyméthane, solvant dans lequel la base forte est stable. Le mélange obtenu est agité pendant quelques heures. Le polymère (I) est lavé sous vide puis séché. Il peut ensuite être utilisé directement sans autre purification.

Le composé de formule (I) dans laquelle R₄ ne représente pas l'atome d'hydrogène, peut être obtenu en faisant réagir le composé (I) correspondant dans lequel R₄ représente l'atome d'hydrogène, avec un composé de formule R₄X' dans laquelle X' représente un atome d'halogène et R₄ est tel que défini ci-dessus mais ne représente pas l'atome d'hydrogène, à une température comprise entre -10 et +10° C, de préférence à 0° C, dans un solvant tel que le THF. Après filtration et lavage, le produit ainsi obtenu peut être traité avec une base forte pour obtenir le composé (I) souhaité.

L'invention a également pour objet, à titre de produits industriels, et notamment à titre de produits industriels destinés à la préparation des produits de formule (I), les produits de formule (3) tels que décrits ci-dessus.

Les composés de formule (1) et (2) sont généralement des produits commerciaux et peuvent être obtenus selon les méthodes classiques connues de l'homme de l'art. Les composés de formule (I') dans laquelle R'₅ représente l'atome d'hydrogène, un radical alkyle inférieur ou alkoxy inférieur, peuvent être préparés selon les méthodes classiques connues de l'homme de l'art (Johnson, A. W., 1993, Series, Editor, *Ylides and Imines of phosphorus*, Wiley & Wiley : New York).

Les exemples suivants sont présentés pour illustrer les procédures ci-dessus.

### PARTIE EXPÉRIMENTALE :

### Exemple 1 : Préparation d'un ylure supporté

### Etape 1a : Préparation du composé (3)

7 g de résine de Merrifield (1 meq/g) sont mis sous atmosphère inerte d'argon. Une solution de 21 mmol de P(NMe₂)₃ dans 100 ml de dichloroéthane est ajoutée et maintenue sous agitation magnétique pendant 5 jours à température ambiante. Le polymère est ensuite lavé plusieurs fois avec du dichlorométhane pour entraîner l'excès de phosphine. Il est ensuite séché sous vide jusqu'à ce que la masse du polymère obtenu ne change plus (8 g). Le composé (3) est obtenu sous forme d'un fin sable blanc. (Pf(dec.) : 325° C).

Le taux de greffage est contrôlé par la réactivité du polymère obtenu. Le solvant utilisé pour le greffage peut aussi être du diméthoxyéthane.

### 1b : préparation du composé (I)

A 1 g du composé (3) en suspension dans 20 ml de tétrahydrofurane (THF) sont ajoutés 1,1 mmol de butyllithium à température ambiante. Le mélange devient rouge et on observe un dégagement de chaleur. L'agitation est maintenue pendant une heure. On rajoute alors 40 ml de THF et on récupère le polymère par filtration. On lave ensuite le polymère avec 40 ml de THF. L'opération est répétée deux fois. On obtient alors une poudre orangée qui est directement utilisée dans les réactions de déprotonation. Après chaque utilisation le polymère précurseur (3) est récupéré par filtration et peut être de nouveau transformé en ylure supporté.

### Exemple 2 : Utilisation des composés selon l'invention dans une réaction de C-alkylation.

### Exemple 2a : C-benzylation de la N-méthyl-2-pyrrolidinone

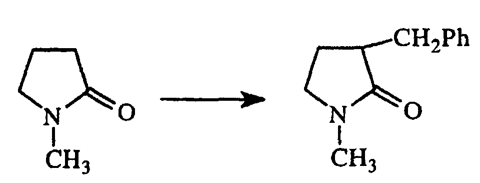

A une solution de 5 mmoles de N-méthyl-2-pyrrolidinone dans 23 ml de THF, on ajoute 6 mmoles de (Me₂N)₃P=C(CH₃)₂ dans 27 ml de THF. La solution est chauffée à 45° C pendant 6 heures puis, 6 mmoles de bromure de benzyle sont additionnées et l'agitation est maintenue une heure supplémentaire à 45° C. On laisse le mélange réactionnel revenir lentement à température ambiante. Après extraction avec 60 ml d'éther, filtration et évaporation du solvant, l'huile obtenue est purifiée sur colonne chromatographique (éluant : éther/méthanol 95/5).

RMN ¹H (CDCl₃) : 2,00 (m, 2H, CH₂) ; 2,63 (m, 2H, CH₂-N) ; 2,79 (s, 3H, CH₃-N) ; 3,10 (m, 3H, CH et CH₂Ph) ; 7,19 (m, 5H, Hₐᵣₒₘ).

RMN ¹³C (CDCl₃) : 23,88 (s, CH₂) ; 29,59 (s, CH₃-N) ; 36,99 (s, CH₂) ; 43,27 (s, CH) ; 47,39 (s, CH₂-N) ; 126,17, 128,28, 128,88 (s, CHₐᵣₒₘ) ; 139,29 (s, Cipₛₒ) ; 175,72 (s, CO).

### Exemple 2b : C-alkylation en position 3 de benzodiazépines selon le schéma suivant

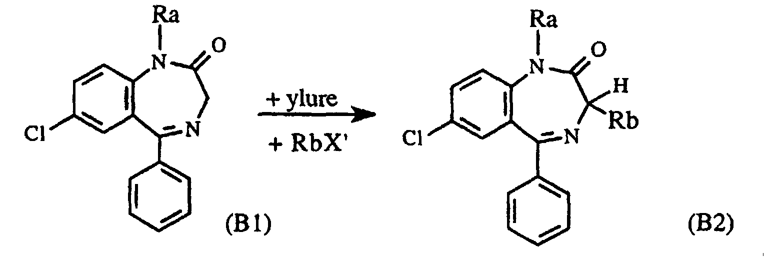

A une solution de 1 mmole du composé (B1) dans 5 ml de THF, on ajoute, à -78° C, 1,5 mmoles d'ylure (Me₂N)3P=C(CH₃)₂ dans 7,5 ml de THF. Le mélange réactionnel est agité pendant 15 minutes puis, 1,5 mmoles du composé de formule RbX' dans laquelle X' représente un atome d'halogène et Rb un radical alkyl éventuellement substitué, sont additionnées. L'agitation est maintenue 30 minutes supplémentaires. Après extraction avec 60 ml d'éther, filtration et évaporation du solvant, l'huile est purifiée sur colonne chromatographique (éluant : pentane/éther 50/50). Le composé de formule (B2) est obtenu sous forme d'une poudre.

Les résultats obtenus selon la valeur de Ra et Rb sont résumés dans le tableau ci-dessous.

| Ex | Ra | Rb | Rendement (%) |
|---|---|---|---|
| 2b₁ | -CH₂Ph | -CH₂Ph | 38 |
| 2b₂ | -CH₂Ph | -CH₂CO₂tBu | 42 |
| 2b₃ | -CH₂Ph | -CH₃ | 67 |
| 2b₄ | -CH₃ | -CH₂Ph | 55 |
| 2b₅ | -CH₃ | -CH₂CO₂tBu | 42 |
| 2b₆ | -CH₃ | -CH₃ | 43 |
| 2b₇ | -CH₂CO₂tBu | -CH₂CO₂tBu | 48 |
| 2b₈ | -CH₂CO₂tBu | -CH₂Ph | 39 |
| 2b₉ | -CH₂CO₂tBu | -CH₃ | 54 |

### Caractéristiques des composés :

### Exemple 2b₁: point de fusion 87° C

RMN ¹H (CDCl₃) : 3,65 (m, 2H, CH₂Ph) ; 3,87 (m, 1H, CH) ; 4,68 (d, ²J_{(HH)}=15,3 Hz, 1H, N-CH₂Ph) ; 5,68 (d, ²J_{(HH)} = 15,3 Hz, 1H, N-CH₂Ph) ; 7,20 (m, 18H, Hₐᵣₒₘ).

RMN ¹³C (CDCl₃) : 37,95 (s, CH-CH₂Ph) ; 49,95 (s, N-CH₂Ph) ; 65,27 (s, CH-CH₂Ph) ; 123,92-131,24 (s, CHₐᵣₒₘ) ; 129,43, 131,99, 136,27, 137,94, 138,99, 140,05 (s, Cq) ; 167,43 ; 169,05 (s, CO et CN).

### Exemple 2b₂: point de fusion 166° C

RMN ¹H (CDCl₃) : 1,47 (s, 9H, CH₃) ; 3,25 (dd, ²J_{(HH)} = 16,9 Hz, ²J_{(HH)} = 7,5 Hz, H_{B}, CH₂) ; 3,43 (dd, ²J_{(HH)} = 16,9 Hz, ²J_{(HH)} = 6,5 Hz, H_{B',} CH₂) ; 4,19 (dd, ³J_{(HH)} = 7,6 Hz, ³J_{(HH)} = 6,5 Hz, H_{A,} CH) ; 4,75 (d, ²J_{(HH)} = 15,5 Hz, H_{A}, N-CH₂Ph) ; 5,60 (d, ²J_{(HH)} = 15,5 Hz, H_{B}, N-CH₂Ph) ; 6,97-7,43 (m, 13H, Hₐᵣₒₘ).

RMN ¹³C (CDCl₃) :28,14 (s, CH₃) ; 37,80 (s, CH₂COOtBu) ; 50,28 (s, N-CH₂Ph) ; 60,62 (s, CH); 80,70 (s, Cq(CH₃)₃) ; 124,01-131,38 (s, CHₐᵣₒₘ) ; 129,54 ; 132,11 ; 136,20 ; 137,90 ; 140,31 (s, Cq) ; 167,50 ; 169,02 ; 171,30 (s, CO, CN et COO).

### Exemple 2b₃: point de fusion 164° C

RMN ¹H (CDCl₃) : 1,77 (d, ³J_{(HH)} = 6,4 Hz, 3H, CH₃) ; 3,81 (q, ³J_{(HH)} = 6,4 Hz, 1H, CH) ; 4,68 (d, ²J_{(HH)} = 15,5 Hz, H_{A}, CH₂Ph) ; 5,70 (d, ²J_{(HH)} = 15,5 Hz, H_{B}, CH₂Ph) ; 6,99-7,42 (m, 13H, Hₐᵣₒₘ).

RMN ¹³C (CDCl₃) : 17,32 (s, CH₃) ; 49,82 (s, CH₂Ph) ; 58,81 (s, CH) ; 123,94-131,30 (s, CHₐᵣₒₘ) ; 130,38 ; 131,07 ; 136,39; 137,93 ; 140,20 (s, Cq) ; 167,16; 170,35 (s, CO et CN).

### Exemple 2b₄: point de fusion 85° C

RMN ¹H (CDCl₃) : 3,40 (s, 3H, N-CH₃) ; 3,56-3,77 (m, 3H, CH et CH₂Ph) ; 7,19-7,55 (m, 13H, Hₐᵣₒₘ).

RMN ¹³C (CDCl₃) : 35,28 (s, N-CH₃) ; 38,10 (s, CH₂Ph) ; 65,27 (s, CH) ; 122,77-131,45 (s, CHₐᵣₒₘ) ; 129,16 ; 130,32 ; 138,15 ; 139,21 ; 142,15 (s, Cq) ; 168,01 ; 170,05 (s, CO et CN).

### Exemple 2b₅ : point de fusion 141° C

RMN ¹H (CDCl₃) : 1,45 (s, 9H, CH₃) ; 3,40 (s, 3H, N-CH₃) ; 3,11 (dd, ²J_{(HH)} = 17,1 Hz, ²J_{(HH)} = 7,0 Hz, H_{B,} CH₂) ; 3,39 (dd, ²J_{(HH)} = 17,1 Hz, ²J_{(HH)} = 6,8 Hz, H_{B'}, CH₂) ; 4,07 (dd, ³J_{(HH)} = 7,0 Hz, ³J_{(HH)} = 6,8 Hz, H_{A}, CH) ; 7,25-7,57 (m, 8H, Hₐᵣₒₘ).

RMN ¹³C (CDCl₃) : 28,12 (s, CH₃) ; 35,21 (s, N-CH₃) ; 37,91 (s, CH₂) ; 60,56 (s, CH) ; 80,59 (s, Cq(CH3)3) ; 122,83-130,36 (s, CHₐᵣₒₘ) ; 131,52 ; 137,97 ; 139,40 ; 142,20 (s, Cq) ; 167,20 ; 169,70 ; 171,25 (s, CO, CN et COO).

### Exemple 2b₆: point de fusion 76° C

RMN ¹H (CDCl₃) : 1,71 (d, ³J_{(HH)} = 6,5 Hz, 3H, CH₃) ; 3,39 (s, 3H, N-CH₃) ; 3,70 (q, ³J_{(HH)} = 6,5 Hz, CH) ; 7,25-7,60 (m, 8H, Hₐᵣₒₘ).

RMN ¹³C (CDCl₃) : 17,43 (s, CH₃) ; 35,15 (s, N-CH₃) ; 58,90 (s, CH) ; 122,59-130,54 (s, CHₐᵣₒₘ) ; 129,11 ; 129,63 ; 131,38 ; 138,13 (s, Cq) ; 166,85 ; 171,21 (s, CO et CN).

### Exemple 2b₇: point de fusion 128° C

RMN ¹H (CDCl₃) : 1,40 (s, 9H, CH₃) ; 1,44 (s, 9H, CH₃) ; 3,14 (dd, ²J_{(HH)} = 16,9 Hz, ²J_{(HH)} = 6,8Hz, 1H, H_{B}) ; 3,43 (dd, ²J_{(HH)} = 16,9 Hz, ²J_{(HH)} = 7,0 Hz, 1H, H_{B'}) ; 4,15 (dd, ³J_{(HH)} = 7,0 Hz, ³J_{(HH)} = 6,8 Hz, 1H, H_{A}) ; 4,20 (d, ²J_{(HH)} = 17,1 Hz, 1H, CH₂COOtBu) ; 4,48 (d, ²J_{(HH)} = 17,1 Hz, 1H, CH₂COOtBu) ; 7,25-7,60 (m, 8H, Hₐᵣₒₘ).

RMN ¹³C (CDCl₃) : 27,90 ; 28,06 (s, CH₃) ; 7,82 (s, CH₂) ; 50,81 (s, N-CH₂) ; 60,33 (s, CH) ; 80,61 ; 82,43 (s, Cq(CH₃)₃) ; 123,02-131,64 (s, CHₐᵣₒₘ) ; 129,89 ; 130,60 ; 130,98 ; 138,08 (s, Cq) ; 67,39; 167,76; 170,92 (s, CO, CN et COO).

### Exemple 2b₈: point de fusion 81° C

RMN ¹H (CDCl₃) : 1,41 (s, 9H, CH₃) ; 3,72 (m, 3H, CH et CH₂Ph) ; 4,40 (d, ²J_{(HH)} = 16,7 Hz, 1H, CH₂COOtBu) ; 4,52 (d, ²J_{(HH)} = 16,7 Hz, 1H, CH₂COOtBu) ; 7,20-7,58 (m, 13H, Hₐᵣₒₘ).

RMN ¹³C (CDCl₃) : 27,79 (s, CH₃) ; 37,69 (s, CH₂Ph) ; 50,77 (s, N-CH₂COOtBu) ; 64,72 (s, CH) ; 82,33 (s, Cq(CH₃)₃) ; 122,69-131,45 (s, CHₐᵣₒₘ) ; 130,68 ; 131,10 ; 138,10 ; 138,91; 141,08 (s, Cq) ; 167,30 ; 167,47 ; 169,49 (s, CO, CN et COO).

### Exemple 2b₉: point de fusion 152° C

RMN ¹H (CDCl₃):1,41 (s, 9H, CH₃) ; 1,70 (d, ³J_{(HH)} = 6,4 Hz, 3H, CH-CH₃) ; 3,77 (q, ³J_{(HH)} = 6,4 Hz, 1H, CH-CH₃) ; 4,23 (d, ²J_{(HH)} = 17,2 Hz, 1H, CH₂COOtBu) ; 4,51 (d, ²J_{(HH)} = 17,2 Hz, 1H, CH₂COOtBu) ; 7,21-7,60 (m, 8H, Hₐᵣₒₘ).

RMN ¹³C (CDCl₃) : 17,16 (s, CH₃) ; 27,81 (s, CH-CH₃) ; 50,65 (s, CH₂) ; 58,46 (s, CH) ; 82,25 (s, Cq(CH₃)₃) ; 122,29-131,37 (s, CHₐᵣₒₘ) ; 129,41 ; 130,82 ; 138,04 ; 141,18 (s, Cq) ; 167,04 ; 167,59; 170,50 (s, CO, CN et COO).

Les exemples 2a et 2b peuvent également être mis en oeuvre en utilisant un ylure supporté tel que défini dans la présente invention, et plus particulièrement avec un polymère de formule (a) tel que définie ci-dessus dans laquelle m > 1.

### Exemple 2c : C-alkylation en position 3 de benzodiazépines selon le schéma suivant

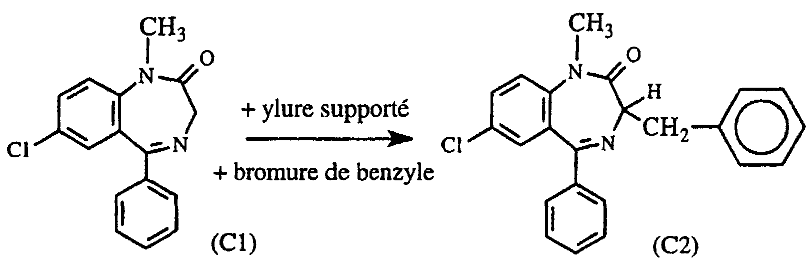

1 g d'ylure supporté tel que préparé selon l'exemple 1, est agité dans 20 ml de THF. On ajoute une solution de 0,7 mmole du composé (C1) dans 10 ml de THF et le mélange réactionnel est maintenu sous agitation pendant une demi-heure. On ajoute alors 1 mmole de bromure de benzyle distillé et on chauffe le mélange réactionnel à 60° C pendant 12 heures. La solution est alors séparée des polymères, les solvants sont évaporés sous vide et le composé (C2), identique au composé B2 de l'exemple 2b4, est récupéré sous forme d'une poudre orange avec un rendement de 20 %.

### Exemple 2d : C-alkylation de benzodiazépines en position 3 à l'aide d'un aldéhyde

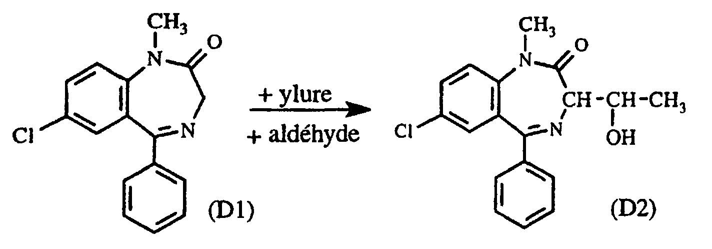

A une solution de 1 mmole du composé (D1) dans 5 ml de THF, on ajoute, à -78° C, 1,5 mmoles d'ylure (Me₂N)₃P=C(CH₃)₂ dans 7,5 ml de THF. Le mélange réactionnel est agité pendant 15 minutes puis, 1,5 mmoles d'éthanal sont additionnées. L'agitation est maintenue 30 minutes supplémentaires. Après extraction avec 60 ml d'éther, filtration et évaporation du solvant, l'huile est purifiée sur colonne chromatographique (éluant pentane/éther 95/5). Le composé de formule (D2) est obtenu sous forme d'une poudre (44 %).

### Exemple 3 : Utilisation des composés selon l'invention dans une réaction de N-alkylation.

### Exemple 3a : N-fonctionnalisation du norvalium

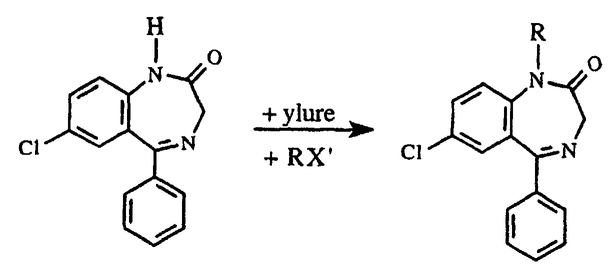

1 g de l'ylure supporté tel que préparé selon l'exemple 1, est agité dans 20 ml de THF. On ajoute une solution de 0,8 mmole de norvalium dans 10 ml de THF et le mélange est maintenu sous agitation pendant une demi-heure. On ajoute alors 1 mmol d'électrophile distillé de formule RX' dans laquelle X' représente un atome d'halogène et R un radical alkyle éventuellement substitué ou SiMe3, et on laisse le mélange sous agitation pendant 4 heures. La solution est alors séparée des polymères, les solvants sont évaporés sous vide et l'huile obtenue est reprise dans les solvants indiqués ci-dessous. Le polymère est quant à lui lavé et peut être réutilisé ultérieurement.

| Ex | RX' | Solvant | Rendement (%) |
|---|---|---|---|
| 3a₁ | Me₃SiCl | Pentane | 80 |
| 3a2 | BrCH₂CO₂tBu | Et₂O | 70 |
| 3a₃ | PhCH₂Br | Et₂O | 76 |

### Exemple 3a₁ :

RMN ¹H (CDCl₃) : 0,30 (s, 9H, CH₃) ; 4,10 (s large, 2H, CH₂) ; 7,30 (m, 8H, Hₐᵣₒₘ).

RMN ¹³C (CDCl₃) : -0,15 (s, CH₃) ; 53,71 (s, CH₂) ; 127,62-128,11 (s, Cq) ; 129,63-130,52 (s, CHₐᵣₒₘ) ; 169,50 ; 169,52 (s, CO et CN).

### Exemple 3a₂:

RMN ¹H (CDCl₃) : 1,40 (s, 9H, CH₃) ; 3,80 (d, ²J_{(HH)} = 10,7 Hz, 1H, CH₂) ; 4,21 (d, ²J_{(HH)} = 15,0 Hz, 1H, CH₂COOtBu) ; 4,45 (d, ²J_{(HH)} = 15,0 Hz, 1H, CH₂COOtBu) ; 4,85 (d; ²J_{(HH)} = 10,7 Hz, 1H, CH₂) ; 7,40 (m, 8H, Hₐᵣₒₘ).

RMN ¹³C (CDCl₃) : 27,65 (s, CH₃) ; 51,03 (s, CH₂-COOtBu) ; 51,03 (s, CH₂-COOtBu) ; 55,88 (s, CH₂) ; 83,50 (s, Cq(CH₃)₃) ; 123,63-131,98 (s, CHₐᵣₒₘ) ; 130,23 ; 130,64 ; 137,85 ; 140,56 (s, Cᵢₚₛₒ) ; 168,30 ; 170,26 ; 170,58 (s, CO, COO et CN).

### Exemple 3a₃ :

RMN ¹H (CDCl₃) : 1,41 (s, 9H, CH₃) ; 3,80 (d, ²J_{(HH)} = 10,5 Hz, 1H, CH₂) ; 4,23 (d, ²J_{(HH)} = 17,2 Hz, 1H, CH₂COOtBu) ; 4,51 (d, ²J_{(HH)} = 17,2 Hz, 1H, CH₂COOtBu) ; 4,96 (d, ²J_{(HH)} = 10,5 Hz, 1H, CH₂) ; 7,21-7,60 (m, 8H, Hₐᵣₒₘ).

RMN ¹³C (CDCl₃) : 17,16 (s, CH₃) ; 27,81 (s, CH-CH₃) ; 50,65 (s, CH₂) ; 58,46 (s, CH) ; 82,25 (s, Cq(CH₃)₃) ; 122,29-131,37 (s, CHₐᵣₒₘ) ; 129,41; 130,82 ; 138,04 ; 141,18 (s, Cq) ; 167,04 ; 167,59 ; 170,50 (s, CO, CN et COO).

### Exemple 3b : N-alkylation de lactames

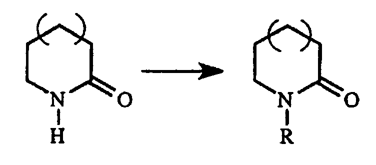

A une solution de 3 mmoles de substrat dans 14 ml de THF, on ajoute lentement, à 25° C, une solution de 3,6 mmoles de (Me₂N)₃P=C(CH₃)₂ dans 17 ml de THF. Le mélange réactionnel est agité à 25° C pendant une heure puis, 3,6 mmoles d'électrophile distillé de formule RX' dans laquelle X' représente un atome d'halogène et R un radical alkyle éventuellement substitué, sont additionnées. L'agitation est maintenue 3 heures supplémentaires. La solution surnageante est séparée des sels de phosphonium formés par extraction avec 60 ml d'éther. Après concentration, l'huile obtenue est purifiée sur colonne chromatographique.

| RX' | Eluant | Rendement (%) |
|---|---|---|
| CH₃I | Et₂O/MeOH 85/15 | 60 |
| BrCH₂CO₂tBu | Et₂O | 70 |
| PhCH₂Br | Et₂O/MeOH 30/70 | 71 |

### Caractérisation de la lactame N-alkylée :

| | |
|---|---|
| R = CH₃- | RMN ¹H (CDCl₃) : 1,82 (m, 4H, CH₂-CH₂) ; 2,47 (m, 2H, CH₂-CO) ; 2,98 (s, 3H, CH₃-N) ; 3,31 (m, 2H, CH₂-N). |
| | |
| | I.R. (CDCl₃) : 1642 cm⁻¹ (CO). |
| | |
| R = tBuOC(O)CH₂- | RMN ¹H (CDCl₃) : 1,43 (s, 9H, CH₃) ; 1,80 (m, 4H, CH₂-CH₂) ; 2,55 (m, 2H, CH₂-CO) ; 3,35 (m, 2H, CH₂-N) ; 4,09 (s, 2H, CH₂-COO). |
| | |
| | RMN ¹³C (CDCl₃) : 20,44 ; 22,42 (s, CH₂) ; 27,73 (s, CH₃) ; 31,72 (s, CH₂-CO) ; 49,21 ; 49,90 (s, CH₂-N) ; 82,49 (s, C(CH₃)₃) ; 168,59 ; 172,37 (s, CO). |
| | |
| R = PhCH₂- | RMN ¹H (CDCl₃) : 1,70 (m, 4H, CH₂-CH₂) ; 2,45 (m, 2H, CH₂-CO) ; 3,14 (m, 2H, CH₂-N) ; 4,53 (s, 2H, CH₂Ph) ; 7,20 (m, 5H, Hₐᵣₒₘ). |
| | |
| | RMN ¹³C (CDCl₃) : 20,70 ; 22,60 (s, CH₂) ; 31,89 (s, CH₂-CO) ; 47,08 ; 49,91 (s, CH₂-N) ; 127,15 ; 127,57 ; 128,37 (s, CHₐᵣₒₘ) ; 136,56 (s, Cᵢₚₛₒ) ; 170,77 (s, CO) |

Cet exemple peut également être mis en oeuvre en utilisant un ylure supporté tel que défini dans la présente invention, et plus particulièrement avec un polymère de formule (a) tel que définie ci-dessus dans laquelle m > 1.

### Exemple 3c : N-benzylation de peptides

La N-t-Boc-L-Leucine et le chlorhydrate de l'ester méthylique de la L-phénylalanine, sont préalablement couplés en présence d'un agent de couplage BrOP afin d'obtenir le dipeptide correspondant.

A une solution d'un équivalent du dipeptide ainsi obtenu (1,60 mmoles) dans 9 ml de THF, on ajoute lentement, à 25° C, 4 équivalents d'ylure (5,85 mmoles) dans 33 ml de THF. Le mélange réactionnel est agité à 25° C pendant deux heures puis, 4 équivalents de bromure de benzyle (5,85 mmoles) sont additionnés. L'agitation est maintenue 3 heures supplémentaires. La solution surnageante est séparée des sels de phosphonium formés par extraction avec 60 ml d'éther. Après concentration, l'huile obtenue fait l'objet d'une purification sur colonne de silice (éluant : éther/hexane 50/50).

Le produit de monobenzylation est obtenu avec un rendement de 12 % et celui de dibenzylation avec un rendement de 28 %. Ces composés ont fait l'objet d'analyses par chromatographie en phase gazeuse couplé spectrométrie de masse impact électronique.

### Exemple 3d : réutilisation d'un ylure supporté pour la N-fonctionnalisation du norvalium

Le polymère de l'exemple 2c est lavé plusieurs fois dans un mélange dichlorométhane /acétonitrile, séché sous vide et réutilisé dans la réaction de N-benzylation du norvalium dans les conditions telles que décrites dans l'exemple 3a. Le composé N-benzylé est récupéré avec un rendement de 79 %, rendement comparable à celui obtenu dans l'exemple 3a₃.

## Revendications

1. Utilisation de produits de formule générale I' 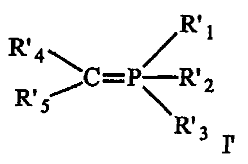 dans laquelle
R'₁, R'₂ et R'₃ représentent, indépendamment, un radical alkoxy en C₁-C₆ ou amino de formule R'R"N dans laquelle R' et R" représentent indépendamment l'un des radicaux non-substitués ou substitués (par un ou plusieurs substituants identiques ou différents) suivants : alkyle en C₁-C₆, alkoxy en C₁-C₆, cycloalkyle hydrocarboné cyclique non aromatique en C₃-C₁₀, aryl-(C₁-C₆)alkyle, aryle, dans lesquels le substituant est un atome de fluor, de chlore, de brome ou d'iode ou un radical alkyle en C₁-C₆, alkoxy en C₁-C₆, cyano, nitro ou di(C₁-C₆alkyl)amino avec les deux radicaux alkyles identiques ou différents, et les radicaux aryles étant insaturés, monocycliques ou constitués de cycles condensés, carbocycliques ou hétérocycliques, étant entendu que les radicaux hétérocycliques peuvent inclure un ou plusieurs hétéroatomes identiques ou différents choisis parmi l'oxygène, le soufre ou l'azote ;
R'₄ représente l'atome d'hydrogène, un radical alkyle en C₁-C₆ ou alkoxy en C₁-C_{6;}
R'₅ représente l'atome d'hydrogène, un radical alkyle en C₁-C₆, alkoxy en C₁-C₆ ou un support polymérique ;
en tant que base forte faiblement nucléophile.

2. Utilisation selon la revendication 1, **caractérisée en ce que** R'₁, R'₂ et R'₃ représentent, indépendamment, un radical amino de formule RR'N.

3. Utilisation selon l'une des revendications 1 à 2, **caractérisée en ce que** R'₁, R'₂ et R'₃ représentent, indépendamment, le radical diméthylamino, (méthyl)(éthyl)amino, diéthylamino.

4. Utilisation selon l'une des revendications 1 à 3, **caractérisée en ce que** R'4 représente l'atome d'hydrogène ou un radical alkyle en C₁-C₆.

5. Utilisation selon l'une des revendications 1 à 4, **caractérisée en ce que** R'4 représente l'atome d'hydrogène ou le radical méthyle ou éthyle.

6. Utilisation selon l'une des revendications 1 à 5, **caractérisée en ce que** R'₅ représente un radical alkyle en C₁-C₆ ou un support polymérique.

7. Utilisation selon l'une des revendications 1 à 6, **caractérisée en ce que** le support polymérique R'₅ est de type méthacrylique, acrylique ou polystyrénique.

8. Utilisation selon l'une des revendications 1 à 7, **caractérisée en ce que** le support polymérique R'₅ est de formule générale (s) 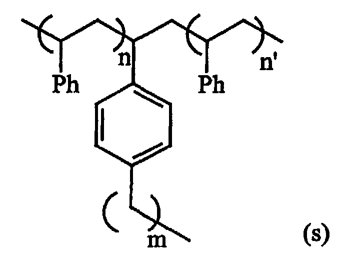 dans laquelle n, n', m sont des entiers supérieurs ou égaux à un.

9. Utilisation selon la revendication 8, **caractérisée en ce que** le support polymérique de formule (s) provient du polymère correspondant de formule (p) 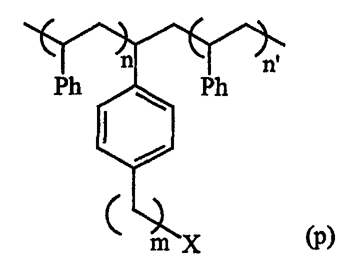 dans laquelle n, n', m sont tels que définis à la revendication 8 et X représente un groupe partant.

10. Utilisation selon la revendication 9, **caractérisée en ce que** X représente l'atome de chlore et m est égal à 1.

11. Utilisation selon la revendication 10, **caractérisée en ce que** le support provient de la résine de Merrifield.

12. Utilisation selon l'une des revendications 1 à 11, **caractérisée en ce que** les produits de formule générale I' répondent aux formules suivantes :
- copolymère styrène/divinylbenzène-tris(diméthylamino)méthylènephosphorane ;
- tris(diméthylamino)-C-diméthylméthylène phosphorane.

13. Utilisation selon l'une des revendications 1 à 12, dans les réactions de N-alkylation.

14. Utilisation selon l'une des revendications 1 à 13, dans les réactions de N-alkylation des lactames, des succinimides, d'oligopeptides et de benzodiazépines.

15. Utilisation selon l'une des revendications 1 à 12, dans les réactions de C-alkylation.

16. Utilisation selon l'une des revendications 1 à 12 et 15, dans les réactions de C-alkylation des lactames, des succinimides, de bases de Schiff et de benzodiazépines.

17. Produits de formule générale I 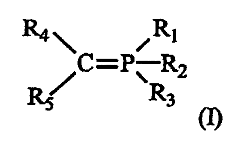 dans laquelle R₁, R₂ et R₃ représentent, indépendamment, un radical alkoxy en C₁-C₆ ou amino de formule R'R"N dans laquelle R' et R" représentent indépendamment l'un des radicaux non-substitués ou substitués (par un ou plusieurs substituants identiques ou différents) suivants : alkyle en C₁-C₆, alkoxy en C₁-C₆, cycloalkyle hydrocarboné cyclique non aromatique en C₃-C₁₀, aryl-(C₁₋C₆)alkyle, aryle, dans lesquels le substituant est un atome de fluor, de chlore, de brome ou d'iode ou un radical alkyle en C₁-C₆, alkoxy en C₁-C₆, cyano, nitro ou di(C₁-C₆alkyl)amino avec les deux radicaux alkyles identiques ou différents, et les radicaux aryles étant insaturés, monocycliques ou constitués de cycles condensés, carbocycliques ou hétérocycliques, étant entendu que les radicaux hétérocycliques peuvent inclure un ou plusieurs hétéroatomes identiques ou différents choisis parmi l'oxygène, le soufre ou l'azote ;
R₄ représente l'atome d'hydrogène, un radical alkyle en C₁-C₆ ou alkoxy en C₁-C₆ ; et
R₅ représente un support polymérique.

18. Produits selon la revendication 17 **caractérisés en ce que** R₁, R₂ et R₃ représentent, indépendamment, un radical amino de formule RR'N.

19. Produits selon l'une des revendications 17 à 18, **caractérisés en ce que** R₁, R₂ et R₃ représentent, indépendamment, le radical diméthylamino, (méthyl)(éthyl)amino ou diéthylamino.

20. Produits selon l'une des revendications 17 à 19, **caractérisés en ce que** R₄ représente l'atome d'hydrogène.

21. Produits selon l'une des revendications 17 à 20, **caractérisés en ce que** le support polymérique R₅ est de type méthacrylique, acrylique ou polystyrénique.

22. Produits selon l'une des revendications 17 à 21 **caractérisés en ce que** le support polymérique de type polystyrénique est de formule générale (s) 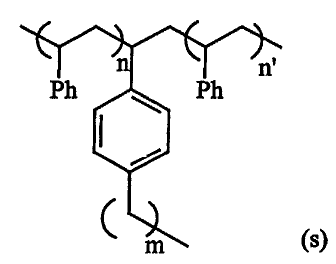 dans laquelle n, n', m sont des entiers supérieurs ou égaux à un.

23. Procédé de préparation des produits selon la revendication 17, **caractérisé en ce que** l'on fait réagir une phosphine de formule générale (1)
PR₁R₂R₃ (1)
dans laquelle R₁, R₂ et R₃ ont la signification indiquée dans la revendication 14, avec un produit de formule générale (2) 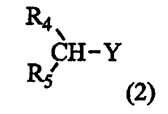 dans laquelle R₄ et R₅ ont la signification indiquée dans la revendication 14 et Y est tel que Y- représente tout anion, pour obtenir le produit de formule générale (3) 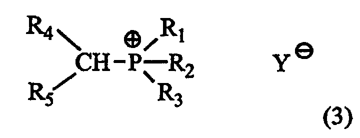 produit (3) ainsi obtenu que l'on traite avec une base forte pour obtenir un produit de formule I.

24. Produits de formule (3) telle que définie à la revendication 23.

## Claims

1. Use of products of general formula I' 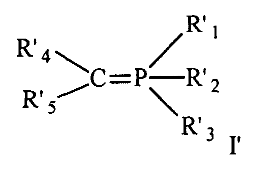 in which
R'₁, R'₂ and R'₃ represent, independently, a C₁-C₆ alkoxy or amino radical of formula R'R"N in which R' and R" represent independently one of the following radicals, non-substituted or substituted (by one or more identical or different substituents) : C₁-C₆ alkyl, C₁-C₆ alkoxy, C₃-C₁₀ non aromatic cyclic hydrocarbon cycloalkyl, aryl-(C₁-C₆)-alkyl, aryl, in which the substituent is a fluorine, chlorine, bromine or iodine atom or a C₁-C₆ alkyl, C₁-C₆ alkoxy, cyano, nitro or di(C₁-C₆alkyl)amino radical where both alkyl radicals are identical or different, and aryl radicals are unsaturated, monocyclic or constituted by condensed, carbocyclic or heterocyclic cycles, given that heterocyclic radicals can include one or several identical or different heteroatoms chosen among oxygen, sulphur or nitrogen;
R'₄ represents the hydrogen atom, a C₁-C₆ alkyl or C₁-C₆ alkoxy radical;
R'₅ represents the hydrogen atom, a C₁-C₆ alkyl radical, C₁-C₆ alkoxy radical or a polymeric support ;
as a slightly nucleophilic strong base.

2. Use according to claim 1, **characterized in that** R'₁, R'₂ and R'₃ represent, independently, an amino radical of formula RR'N.

3. Use according to one of claims 1 to 2, **characterized in that** R'₁, R'₂ and R'₃ represent, independently, the dimethylamino, (methyl)(ethyl)amino, diethylamino radical.

4. Use according to one of claims 1 to 3, **characterized in that** R'₄ represents the hydrogen atom or a C₁-C₆ alkyl radical.

5. Use according to one of claims 1 to 4, **characterized in that** R'₄ represents the hydrogen atom or the methyl or ethyl radical.

6. Use according to one of claims 1 to 5, **characterized in that** R'₅ represents a C₁-C₆ alkyl radical or a polymeric support.

7. Use according to one of claims 1 to 6, **characterized in that** the polymeric support R'₅ is of methacrylic, acrylic or polystyrenic type.

8. Use according to one of claims 1 to 7, **characterized in that** the polymeric support R'₅ is of general formula (s) 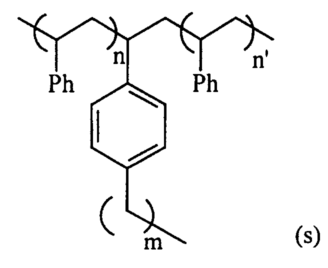 in which n, n', m are integers greater than or equal to one.

9. Use according to claim 8, **characterized in that** the polymeric support of formula (s) originates from the corresponding polymer of formula (p) 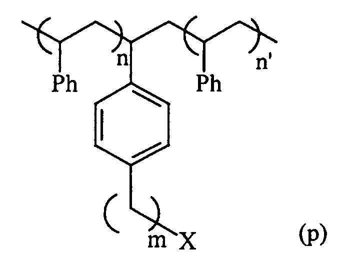 in which n, n', m are as defined in claim 8 and X represents a parting group.

10. Use according to claim 9, **characterized in that** X represents a chlorine atom and m is equal to 1.

11. Use according to claim 10, **characterized in that** the support originates from Merrifield resin.

12. Use according to one of claims 1 to 11, **characterized in that** the products of general formula I' correspond to the following formulae :
- styrene/divinylbenzene-tris(dimethylamino)methylenephosphorane copolymer;
- tris(dimethylamino)-C-dimethylmethylene phosphorane.

13. Use according to one of claims 1 to 12, in N-alkylation reactions.

14. Use according to one of claims 1 to 13, in N-alkylation reactions of lactames, succinimides, oligopeptides and benzodiazepines.

15. Use according to one of claims 1 to 12, in C-alkylation reactions.

16. Use according to one of claims 1 to 12 and 15, in C-alkylation reactions of lactames, succinimides, Schiff bases and benzodiazepines.

17. Products of general formula I 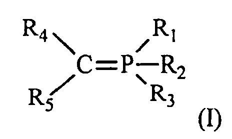 in which R₁, R₂ and R₃ represent, independently, a C₁-C₆ alkoxy or amino radical of formula R'R"N in which R' and R" represent independently one of the following radicals, non-substituted or substituted (by one or more substituents identical or different) : C₁-C₆ alkyl, C₁-C₆ alkoxy, C₃-C₁₀ non aromatic cyclic hydrocarbon cycloalkyl, aryl-(C₁-C₆)-alkyl, aryl, in which the substituent is a fluorine, chlorine, bromine or iodine atom or a C₁-C₆ alkyl, C₁-C₆ alkoxy, cyano, nitro or di(C₁-C₆alkyl)amino radical where both alkyl radicals are identical or different, and aryl radicals are unsaturated, monocyclic or constituted by condensed, carbocyclic or heterocyclic cycles, given that heterocyclic radicals can include one or several identical or different heteroatoms chosen among oxygen, sulphur or nitrogen ;
R₄ represents the hydrogen atom, a C₁-C₆ alkyl or C₁-C₆ alkoxy radical ; and
R₅ represents a polymeric support.

18. Products according to claim 17 **characterized in that** R₁, R₂ and R₃ represent, independently, an amino radical of formula RR'N.

19. Products according to one of claims 17 to 18, **characterized in that** R₁, R₂ and R₃ represent, independently, the dimethylamino, (methyl)(ethyl)amino or diethylamino radical.

20. Products according to one of claims 17 to 19, **characterized in that** R₄ represents the hydrogen atom.

21. Products according to one of claims 17 to 20, **characterized in that** the polymeric support R₅ is of methacrylic, acrylic or polystyrenic type.

22. Products according to one of claims 17 to 21 **characterized in that** the polymeric support of polystyrenic type is of general formula (s) 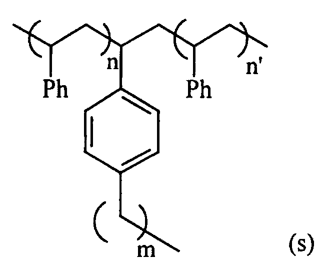 in which n, n', m are of the integers greater than or equal to one.

23. Process for the preparation of products according to claim 17, **characterized in that** a phosphine of general formula (1)
PR₁R₂R₃ (1)
in which R₁, R₂ and R₃ have the meaning indicated in claim 14, is reacted with a product of general formula (2) 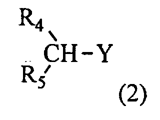 in which R₄ and R₅ have the meaning indicated in claim 14 and Y is such that Y⁻ represents any anion, in order to obtain the product of general formula (3) 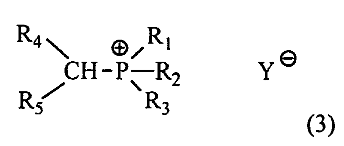 which product (3) thus obtained is treated with a strong base in order to obtain a product of formula I.

24. Products of formula (3) as defined in claim 23.

## Patentansprüche

1. Verwendung von Produkten der allgemeinen Formel I' 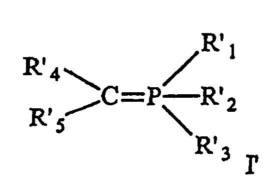 in der
R'₁, R'₂ und R'₃ unabhängig voneinander einen C₁-C₆-Alkoxyrest oder einen Aminorest der Formel R'R"N darstellen, in der R' und R" unabhängig voneinander einen der folgenden nichtsubstituierten oder (durch einen oder mehrere gleiche oder verschiedene Substituenten) substituierten Reste darstellen: C₁-C₆-Alkyl, C₁-C₆-Alkoxy, cyclisches nichtaromatisches C₃-C₁₀-Kohlenwasserstoff-Cycloalkyl, Aryl-(C₁-C₆)-alkyl, Aryl, in denen der Substituent ein Fluor-, Chlor-, Brom- oder Iodatom oder einer der Reste C₁-C₆-Alkyl, C₁-C₆-Alkoxy, Cyano, Nitro oder di(C₁-C₆-Alkyl)amino ist, wobei die beiden Alkylreste gleich oder verschieden sind und die Arylreste ungesättigt sind, monocyclisch sind oder aus kondensierten Ringen bestehen, carbocyclisch oder heterocyclisch sind, wobei die heterocyclischen Reste ein oder mehrere gleiche oder verschiedene Heteroatome, die aus Sauerstoff, Schwefel oder Stickstoff ausgewählt sind, einschließen können;
R'₄ ein Wasserstoffatom, einen C₁-C₆-Alkylrest oder einen C₁-C₆-Alkoxyrest darstellt;
R'₅ ein Wasserstoffatom, einen C₁-C₆-Alkylrest, einen C₁-C₆-Alkoxyrest oder einen polymeren Träger darstellt;
in Form von schwach nukleophilen starken Basen.

2. Verwendung nach Anspruch 1, **dadurch gekennzeichnet, daß** R'₁, R'₂ und R'₃ unabhängig voneinander einen Aminorest der Formel RR'N darstellen.

3. Verwendung nach einem der Ansprüche 1 bis 2, **dadurch gekennzeichnet, daß** R'₁, R'₂ und R'₃ unabhängig voneinander einen der Reste Dimethylamino, (Methyl)(ethyl)amino, Diethylamino darstellen.

4. Verwendung nach einem der Ansprüche 1 bis 3, **dadurch gekennzeichnet, daß** R'₄ ein Wasserstoffatom oder einen C₁-C₆-Alkylrest darstellt.

5. Verwendung nach einem der Ansprüche 1 bis 4, **dadurch gekennzeichnet, daß** R'₄ ein Wasserstoffatom oder einen Methyl- oder Ethylrest darstellt.

6. Verwendung nach einem der Ansprüche 1 bis 5, **dadurch gekennzeichnet, daß** R'₅ einen C₁-C₆-Alkylrest oder einen polymeren Träger darstellt.

7. Verwendung nach einem der Ansprüche 1 bis 6, **dadurch gekennzeichnet, daß** der polymere Träger R'₅ vom Typ Methacryl, Acryl oder Polystyrol ist.

8. Verwendung nach einem der Ansprüche 1 bis 7, **dadurch gekennzeichnet, daß** der polymere Träger R'₅ der allgemeinen Formel (s) 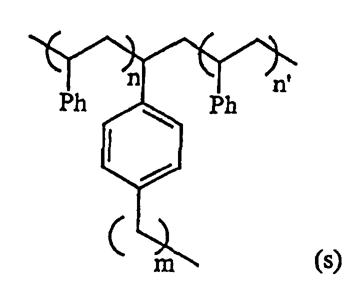 ist, in der n, n', m ganze Zahlen gleich oder größer 1 sind.

9. Verwendung nach Anspruch 8, **dadurch gekennzeichnet, daß** der polymere Träger der Formel (s) von dem entsprechenden Polymer der Formel (p) 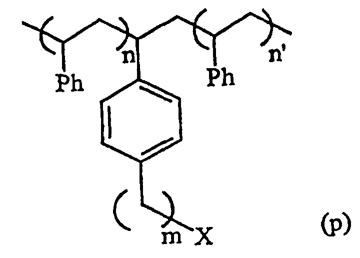 abgeleitet ist, in der n, n', m die in Anspruch 8 angegebene Bedeutung haben und X eine Abgangsgruppe darstellt.

10. Verwendung nach Anspruch 9, **dadurch gekennzeichnet, daß** X ein Chloratom darstellt und m gleich 1 ist.

11. Verwendung nach Anspruch 10, **dadurch gekennzeichnet, daß** der Träger von einem Merrifield-Harz abgeleitet ist.

12. Verwendung nach einem der Ansprüche 1 bis 11, **dadurch gekennzeichnet, daß** die Produkte der allgemeinen Formel I' den folgenden Formeln entsprechen:
- Copolymer Styrol/Divinylbenzol-tris(dimethylamino) methylenphosphoran;
- Tris(dimethylamino)-C-dimethylmethylenphosphoran.

13. Verwendung nach einem der Ansprüche 1 bis 12 bei N-Alkylierungsreaktionen.

14. Verwendung nach einem der Ansprüche 1 bis 13 bei Reaktionen der N-Alkylierung von Lactamen, Succinimiden, Oligopeptiden und Benzodiazepinen.

15. Verwendung nach einem der Ansprüche 1 bis 12 bei C-Alkylierungsreaktionen.

16. Verwendung nach einem der Ansprüche 1 bis 12 und 15 bei Reaktionen der C-Alkylierung von Lactamen, Succinimiden, Schiffschen Basen und Benzodiazepinen.

17. Produkte der allgemeinen Formel I 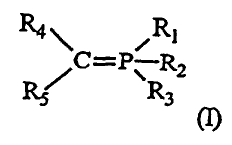 in der R₁, R₂ und R₃ unabhängig voneinander einen C₁-C₆-Alkoxyrest oder einen Aminorest der Formel R'R"N darstellen, in der R' und R" unabhängig voneinander einen der folgenden nichtsubstituierten oder (durch einen oder mehrere gleiche oder verschiedene Substituenten) substituierten Reste darstellen: C₁-C₆-Alkyl, C₁-C₆-Alkoxy, cyclisches nichtaromatisches C₃-C₁₀-Kohlenwasserstoff-Cycloalkyl, Aryl-(C₁-C₆)-alkyl, Aryl, in denen der Substituent ein Fluor-, Chlor-, Brom- oder Iodatom oder einer der Reste C₁-C₆-Alkyl, C₁-C₆-Alkoxy, Cyano, Nitro oder di(C₁-C₆-Alkyl)amino ist, wobei die beiden Alkylreste gleich oder verschieden sind und die Arylreste ungesättigt sind, monocyclisch sind oder aus kondensierten Ringen bestehen, carbocyclisch oder heterocyclisch sind, wobei die heterocyclischen Reste ein oder mehrere gleiche oder verschiedene Heteroatome, die aus Sauerstoff, Schwefel oder Stickstoff ausgewählt sind, einschließen können;
R'₄ ein Wasserstoffatom, einen C₁-C₆-Alkylrest oder einen C₁-C₆-Alkoxyrest darstellt; und
R₅ einen polymeren Träger darstellt.

18. Produkte nach Anspruch 17, **dadurch gekennzeichnet, daß** R₁, R₂ und R₃ unabhängig voneinander einen Aminorest der Formel RR'N darstellen.

19. Produkte nach einem der Ansprüche 17 bis 18, **dadurch gekennzeichnet, daß** R₁, R₂ und R₃ unabhängig voneinander einen der Reste Dimethylamino, (Methyl)(ethyl)amino oder Diethylamino darstellen.

20. Produkte nach einem der Ansprüche 17 bis 19, **dadurch gekennzeichnet, daß** R₄ ein Wasserstoffatom darstellt.

21. Produkte nach einem der Ansprüche 17 bis 20, **dadurch gekennzeichnet, daß** der polymere Träger R₅ vom Typ Methacryl, Acryl oder Polystyrol ist.

22. Produkte nach einem der Ansprüche 17 bis 21, **dadurch gekennzeichnet, daß** der polymere Träger vom Typ Polystyrol der allgemeinen Formel (s) 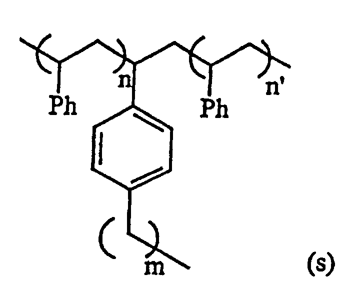 ist, in der n, n', m ganze Zahlen gleich oder größer 1 sind.

23. Verfahren zur Herstellung der Produkte nach Anspruch 17, **dadurch gekennzeichnet, daß** man ein Phosphin der allgemeinen Formel (1)
PR₁R₂R₃ (1),
in der R₁, R₂ und R₃ die in Anspruch 14 angegebene Bedeutung haben, mit einem Produkt der allgemeinen Formel (2) 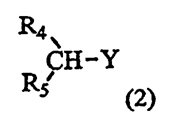 in der R₄ und R₅ die in Anspruch 14 angegebene Bedeutung haben und Y so beschaffen ist, daß Y⁻ irgendein Anion darstellt, reagieren läßt, um das Produkt der allgemeinen Formel (3) 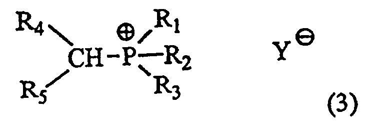 zu erhalten, das man mit einer starken Base behandelt, um ein Produkt der Formel I zu erhalten.

24. Produkte der Formel (3) gemäß Anspruch 23.
